# EUROPEAN PATENT SPECIFICATION

(11) **EP 3 612 107 B1**
(45) Date of publication and mention of the grant of the patent: **12.02.2025**
(21) Application number: 18722863.0
(22) Date of filing: 19.04.2018
(51) Int. Cl.: A61B 17/12

(54) **VARIABLE LENGTH VASCULAR OCCLUSION SYSTEM**
GEFÄSSVERSCHLUSSSYSTEM MIT VARIABLER LÄNGE
SYSTÈME D'OCCLUSION VASCULAIRE À LONGUEUR VARIABLE

(30) Priority: 19.04.2017 US 201762487269 P
(43) Date of publication of application: 26.02.2020
(73) Proprietor: Boston Scientific Scimed, Inc., Maple Grove, MN 55311 (US)
(72) Inventor: TASSONI, Nicholas L., Andover Minnesota 55304 (US); LE, Khoi, Edina Minnesota 55436 (US); JOHNSON, Jeffry D., Crystal Minnesota 55427 (US)
(74) Representative: Vossius & Partner Patentanwälte Rechtsanwälte mbB
(86) International application number: PCT/US2018/028240
(87) International publication number: WO 2018/195236

(56) References cited:
- WO-A1-2007/047111
- US-A- 5 312 415
- US-A1- 2012 053 614
- US-A1- 2015 335 333
- US-A1- 2016 008 003

## Description

### Technical Field

The present disclosure pertains to medical devices and methods for manufacturing and/or using medical devices. More particularly, the present disclosure pertains to configurations of a vascular occlusion system having variable lengths.

### Background

A wide variety of intracorporeal medical devices have been developed for medical use, for example, surgical and/or intravascular use. Some of these devices include guidewires, catheters, medical device delivery systems (e.g., for stents, grafts, replacement valves, etc.), and the like. These devices are manufactured by any one of a variety of different manufacturing methods and may be used according to any one of a variety of methods. There is an ongoing need to provide alternative medical devices as well as alternative methods for manufacturing and/or using medical devices.

WO 2007/047111 A1 relates to an embolic coil delivery system including a catheter. An embolic coil assembly is disposed within the catheter and includes a first embolic coil and a second embolic coil. The first embolic coil has an engaging member at its proximal end and the second embolic coil has an engaging member at its distal end and an engaging member at its proximal end. A pusher wire has an engaging member at its distal end. The embolic coil assembly further may include a control wire, where a coil is engaged with a pusher wire by engaging members in combination with the control wire. Each engaging member includes a ramped face with a slot and a longitudinally-extending passageway for receiving the control wire. The control wire extends through lumens in the pusher wire and in the coil, respectively. The engaging members cannot disengage until the control wire is pulled out of an opening of the passageway. At least one embolic coil differs from at least another embolic coil in at least one coil parameter and the coil parameter can be selected from length, inner diameter, outer diameter, stiffness, secondary shape, and degree of fiber coverage.

US 5,312,415 A relates to a device for delivering embolic coils to a selected site within the vasculature of the human body via use of a catheter. A pusher sheath within the catheter lumen pushes embolic coils mounted on a guidewire through the end of the catheter lumen. The catheter may have a constricted distal tip or other means of controlling the release of the embolic coils. The guidewire may engage the embolic coils from their interior to allow precise placement of the coils.

US 2015/0335333 A1 relates to a medical implant deployment system including a delivery catheter, a delivery system, and an embolic coil. The delivery system includes an elongate release member positioned within the lumen of the catheter.

US 2012/053614 A1 discloses a vascular dilator including first, second, and third markers, respectively, adjacent the proximal end portion thereof. The markers can be in the form of color-coded bands, grooves, or ridges, to provide the surgeon or other medical professional a visual or tactile indicator of the positions of first and second inflatable members, relative to the open distal end portion of an introducer sheath. In other words, the positions and the number of the markers are selected so as to indicate the positions of the inflatable members in a blood vessel, and more specifically relative to the distal end portion of the introducer sheath.

### Summary

The present invention is defined by the features of the independent claim. Further embodiments are provided by the dependent claims.

In a further aspect, a method of embolizing an artery not forming part of the claimed invention may comprise: advancing a vascular occlusion system to a treatment site within the artery, the vascular occlusion system including a plurality of occlusive medical devices releasably connected to a distal end of an elongate shaft and a release wire slidably disposed within a lumen of the elongate shaft and at least a portion of each of the plurality of occlusive medical devices; withdrawing the release wire proximally to release one of the plurality of occlusive medical devices from the elongate shaft at the treatment site; positioning a distal end of one of the plurality of occlusive medical devices still connected to the elongate shaft adjacent the released occlusive medical device; and withdrawing the release wire further proximally to release the positioned occlusive medical device from the elongate shaft.

The above summary of some embodiments, aspects, and/or examples is not intended to describe each embodiment or every implementation of the present disclosure. The figures and the detailed description which follows more particularly exemplify these embodiments.

### Brief Description of the Drawings

The disclosure may be more completely understood in consideration of the following detailed description of various embodiments in connection with the accompanying drawings, in which:
FIG. 1 is a perspective view of an example vascular occlusion system;
FIG. 2 is a partial cut-away view of an example vascular occlusion system;
FIG. 3 illustrates an example attachment mechanism of an example vascular occlusion system;
FIGS. 4-5 are partial cut-away views illustrating actuation of a portion of an example vascular occlusion system;
FIGS. 6-7 illustrate actuation of an example attachment mechanism of an example vascular occlusion system; and
FIG. 8 illustrates the release of multiple portions of an example vascular occlusion system within a vasculature.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described.

### Detailed Description

The following description should be read with reference to the drawings, which are not necessarily to scale, wherein like reference numerals indicate like elements throughout the several views. The detailed description and drawings are intended to illustrate but not limit the claimed invention. Those skilled in the art will recognize that the various elements described and/or shown may be arranged in various combinations and configurations without departing from the scope of the disclosure. The detailed description and drawings illustrate example embodiments of the claimed invention.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosed invention are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

The term "extent" may be understood to mean a greatest measurement of a stated or identified dimension. For example, "outer extent" may be understood to mean a maximum outer dimension, "radial extent" may be understood to mean a maximum radial dimension, "longitudinal extent" may be understood to mean a maximum longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

Diseases and/or medical conditions that impact and/or are affected by the cardiovascular system are prevalent throughout the world. For example, some forms of arterial venous malformations (AVMs) may "feed" off of normal blood flow through the vascular system. Without being bound by theory, it is believed that it may be possible to treat, at least partially, arterial venous malformations and/or other diseases or conditions by starving them of normal, oxygen and/or nutrient-rich blood flow, thereby limiting their ability to grow and/or spread. Other examples of diseases or conditions that may benefit from vascular occlusion include, but are not limited to, bleeds, aneurysms, venous insufficiency, shutting off blood flow prior to organ resection, or preventing embolic bead reflux into branch vessels in the liver. Disclosed herein are medical devices that may be used within a portion of the cardiovascular system in order to treat and/or repair some arterial venous malformations and/or other diseases or conditions. The devices disclosed herein may also provide a number of additional desirable features and benefits as described in more detail below.

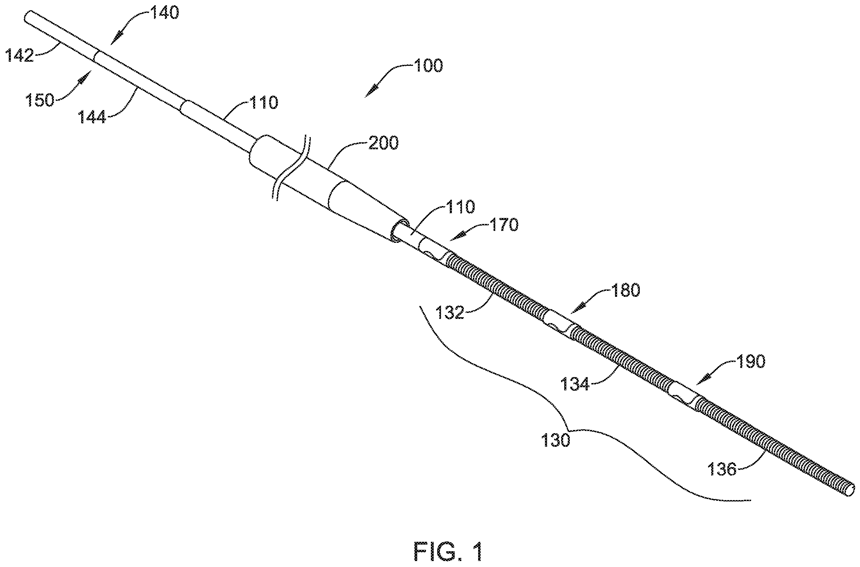

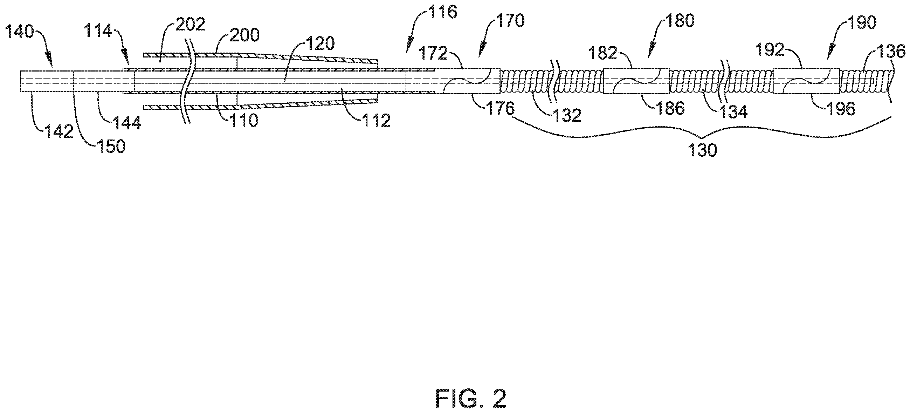

FIGS. 1 and 2 illustrate aspects of an example vascular occlusion system 100. The vascular occlusion system 100 includes an elongate shaft 110 having a lumen 112 extending from a proximal end 114 of the elongate shaft 110 to a distal end 116 of the elongate shaft 110. In some embodiments, the elongate shaft 110 may be a catheter, a hypotube, or other similar tubular structure. Some suitable but non-limiting materials for the elongate shaft 110, for example metallic materials, polymer materials, composite materials, etc., are described below.

The vascular occlusion system 100 includes a microcatheter 200 sized and configured to navigate a vasculature and/or to deliver a plurality of occlusive medical devices 130 to a treatment site within the vasculature, for example an artery or a vein. The elongate shaft 110 and the plurality of occlusive medical devices 130 are slidably disposed within a lumen 202 of the microcatheter 200. In some embodiments, the microcatheter 200 may facilitate percutaneous delivery of the plurality of occlusive medical devices 130 to the vasculature and/or the treatment site. Some suitable but non-limiting materials for the microcatheter 200, for example metallic materials, polymer materials, composite materials, etc., are described below.

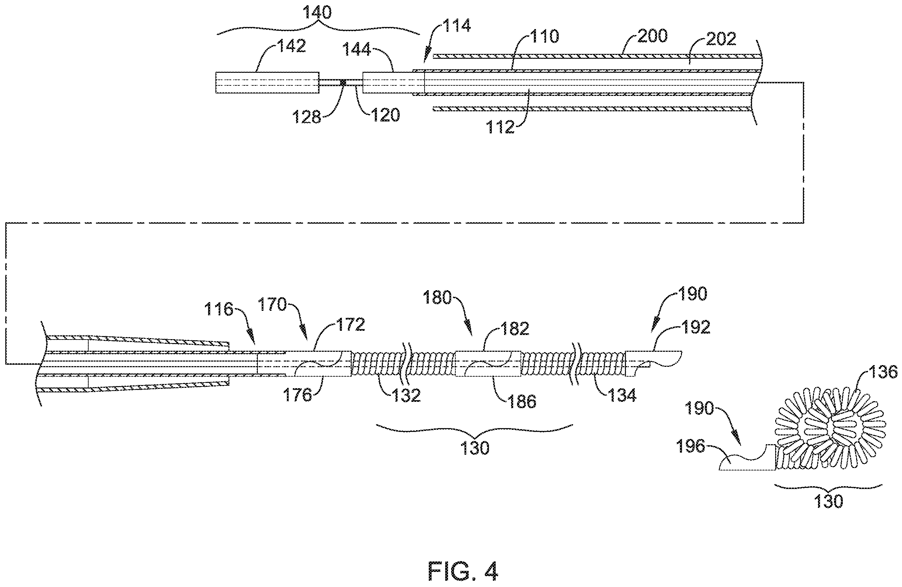

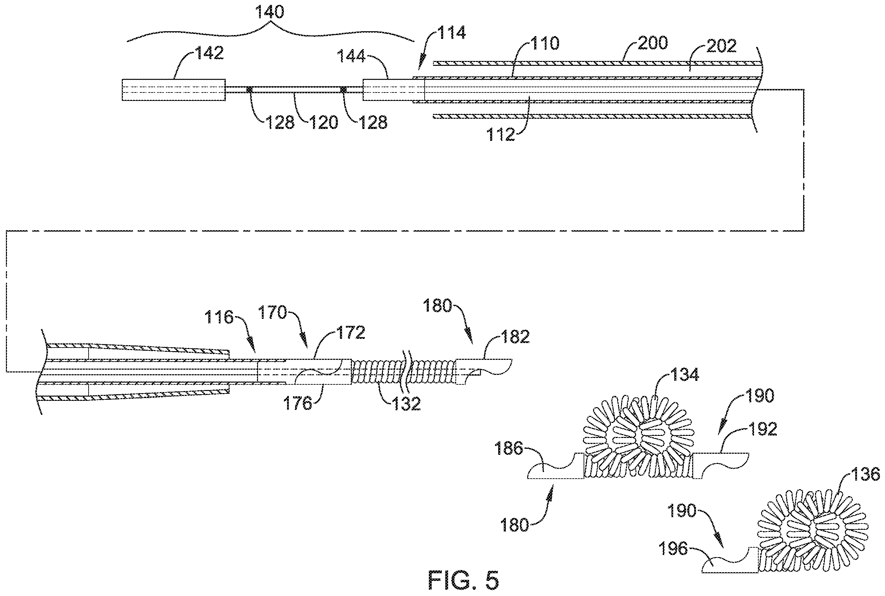

The vascular occlusion system 100 includes the plurality of occlusive medical devices 130 releasably connected to the elongate shaft 110. In some embodiments, the plurality of occlusive medical devices 130 may include a first occlusive medical device 132, a second occlusive medical device 134, and/or a third occlusive medical device 136. Fewer and/or additional occlusive medical devices may be used and/or added as desired. For simplicity, the plurality of occlusive medical devices 130 is illustrated herein as a plurality of shape memory embolic coils, such as those used to treat aneurysms for example, but other suitable occlusive medical devices transported, delivered, used, released etc. in a similar manner are also contemplated, including but not limited to stents, embolic filters, replacement heart valves, occlusion devices, and/or other medical implants, etc. In some embodiments, at least one of the plurality of occlusive medical devices 130 is a coiled member configured to assume a first shape when connected to the elongate shaft 110 and a second shape when disconnected from the elongate shaft 110. At least one of (e.g., one, two, each, etc.) of the plurality of occlusive medical devices 130 may be configured to shift between an elongated delivery configuration (for example, when connected to the elongate shaft 110) as seen in FIGS. 1 and 2, and a deployed configuration (for example, when disconnected from the elongate shaft 110) as seen in FIGS. 4 and 5. In some embodiments, each of the plurality of occlusive medical devices 130 may be configured to assume a different shape after being released from the elongate shaft 110. Turning back to FIGS. 1 and 2, the plurality of occlusive medical devices 130 may be disposed proximate the distal end 116 of the elongate shaft 110 when connected to the elongate shaft 110. In some embodiments, the plurality of occlusive medical devices 130 may be releasably connected to the elongate shaft 110 in series.

The first occlusive medical device 132 may have a proximal end disposed adjacent the distal end 116 of the elongate shaft 110, and the second occlusive medical device 134 may have a proximal end disposed adjacent a distal end of the first occlusive medical device 132. The third occlusive medical device 136 may have a proximal end disposed adjacent a distal end of the second occlusive medical device 134. In at least some embodiments, in the elongated delivery configuration, at least one of the plurality of occlusive medical devices 130 may have a different overall length than a different one of the plurality of occlusive medical devices 130. For example, in the elongated delivery configuration, the third occlusive medical device 136 may be longer than the first occlusive medical device 132 and/or the second occlusive medical device 134. In some embodiments, in the elongated delivery configuration, each of the plurality of occlusive medical devices 130 may have a different overall length. In some embodiments, in the elongated delivery configuration, each of the plurality of occlusive medical devices 130 may have a similar overall length.

At least one of the plurality of occlusive medical devices 130 may have a different stiffness than a different one of the plurality of occlusive medical devices 130. More specifically, a distalmost one of the plurality of occlusive medical devices 130 (e.g., the third occlusive medical device 136) has a stiffness greater than any other of the plurality of occlusive medical devices 130 (e.g., the first occlusive medical device 132 and/or the second occlusive medical device 134), to facilitate anchoring against and/or apposition to a wall at the treatment site (e.g., a wall of a vessel lumen), for example. In some embodiments, a proximalmost one of the plurality of occlusive medical devices 130 (e.g., the first occlusive medical device 132) may have a stiffness less than any other of the plurality of occlusive medical devices 130 (e.g., the second occlusive medical device 134 and/or the third occlusive medical device 136), to facilitate packing against the second occlusive medical device 134 and/or the third occlusive medical device 136, for example. Differences in stiffness may facilitate positioning, placement, fixation, retention, and/or occlusion of the plurality of occlusive medical devices 130 within the vasculature and/or the treatment site. In some embodiments, a distal tip of the distalmost one of the plurality of occlusive medical devices 130 (e.g., the third occlusive medical device 136) may have an atraumatic tip, feature, or distal end. Some suitable but non-limiting materials for the plurality of occlusive medical devices 130, for example metallic materials, polymer materials, composite materials, shape memory materials, etc., are described below.

In some embodiments, one or more (e.g., one, two, each, etc.) of the plurality of occlusive medical devices 130 may include a plurality of fibers and/or a fabric or woven material disposed within and/or attached to individual coils of the plurality of occlusive medical devices 130. The plurality of fibers and/or the fabric or woven material disposed within, attached to, and or embedded within individual coils of the plurality of occlusive medical devices 130 may be configured to enhance coagulation and/or occlusion of the vasculature (e.g., the artery, vein, etc.) and/or the treatment site.

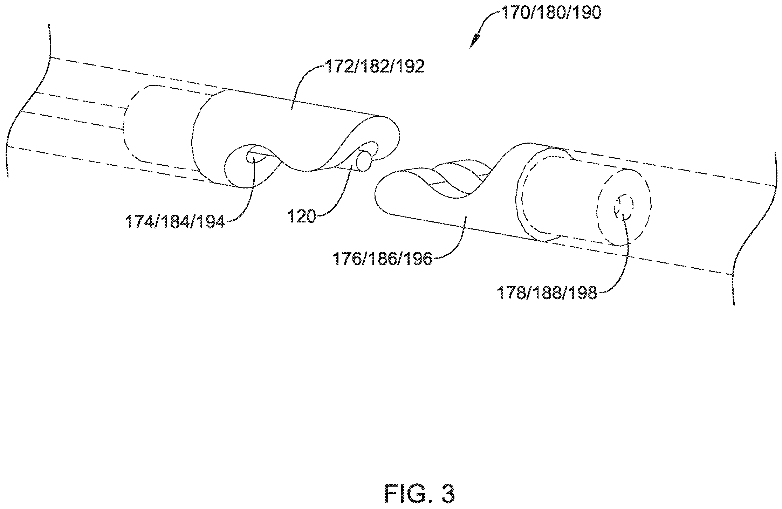

The vascular occlusion system 100 may further comprise one or more attachment mechanisms releasably connecting, attaching, and/or securing the plurality of occlusive medical devices 130 to the distal end 116 of the elongate shaft 110. In some embodiments, the vascular occlusion system 100 may comprise a first attachment mechanism 170 disposed between the distal end 116 of the elongate shaft 110 and the proximal end of the first occlusive medical device 132, wherein the first attachment mechanism comprises a first part 172 and a second part 176. In some embodiments, the vascular occlusion system 100 may comprise a second attachment mechanism 180 disposed between the distal end of the first occlusive medical device 132 and the proximal end of the second occlusive medical device 134, wherein the second attachment mechanism comprises a third part 182 and a fourth part 186. In some embodiments, the vascular occlusion system 100 may comprise a third attachment mechanism 190 disposed between the distal end of the second occlusive medical device 134 and the proximal end of the third occlusive medical device 136, wherein the third attachment mechanism comprises a fifth part 192 and a sixth part 196. Additional details related to the first, second, and third attachment mechanisms will be described below with respect to FIG. 3.

Continuing with FIGS. 1 and 2, the vascular occlusion system 100 includes a release wire 120 slidably disposed within the lumen 112 of the elongate shaft 110 and at least a portion of each of the plurality of occlusive medical devices 130. The release wire 120 is configured to releasably secure and/or attach the plurality of occlusive medical devices 130 to the distal end 116 of the elongate shaft 110 when the release wire 120 is disposed within at least a portion of each of the plurality of occlusive medical devices 130. The release wire 120 extends completely through at least one of the plurality of occlusive medical devices 130. In some embodiments, the release wire 120 may be alternately and/or interchangeably referred to as a pull wire, an actuation wire, and/or a locking wire. The release wire 120 may generally be a solid wire or shaft, but may also be tubular in some embodiments. Some suitable but non-limiting materials for the release wire 120, for example metallic materials, polymer materials, composite materials, etc., are described below.

The vascular occlusion system 100 may include a securement member 140 fixedly attached to and/or extending proximally from the proximal end 114 of the elongate shaft 110, and fixedly attached to a proximal portion and/or a proximal end of the release wire 120. The securement member 140 may include a proximal portion 142, a distal portion 144, and a wall extending from a proximal end of the securement member 140 to a distal end of the securement member 140. In at least some embodiments, the proximal portion 142 of the securement member 140 may be integrally formed with the distal portion 144 of the securement member 140 as a single unitary structure. Some suitable but non-limiting materials for the securement member 140, for example metallic materials, polymer materials, composite materials, etc., are described below.

In some embodiments, the proximal portion 142 of the securement member 140 may be configured to disengage from the distal portion 144 of the securement member 140. The proximal portion 142 of the securement member 140 may be fixedly attached to the proximal portion and/or the proximal end of the release wire 120. The distal portion 144 of the securement member 140 may be fixedly attached to the proximal end 114 of the elongate shaft 110. In at least some embodiments, an outer surface of the distal portion 144 of the securement member 140 may be fixedly attached to an inner surface of the elongate shaft 110 (e.g., a surface defining the lumen 112). Alternatively, in some embodiments, an inner surface of the distal portion 144 of the securement member 140 may be fixedly attached to an outer surface of the elongate shaft 110. In some embodiments, the proximal portion 142 of the securement member 140 may be releasably secured to and/or configured to disengage from the distal portion 144 of the securement member 140 at a joint 150 (e.g., a perforation, a frangible link, etc.) formed in the wall of the securement member 140.

In at least some embodiments, the joint 150 may include a series of apertures extending through the wall of the securement member 140. In some embodiments, the joint 150 may extend circumferentially about an entire circumference of the wall of the securement member 140. In some embodiments, the joint 150 may extend partially and/or intermittently about the entire circumference of the wall of the securement member 140. While an exemplary series of apertures may be round holes, the skilled person will recognize that other suitable shapes (e.g., square, rectangular, ovoid, irregular, etc.) may also be used. For example, in some embodiments, the joint 150 may include a series of rectangular notches having a major dimension oriented circumferentially, the series of rectangular notches extending through the wall of the securement member 140. Additionally, while the joint 150 is illustrated as being generally oriented and/or positioned within a plane perpendicular to a longitudinal axis of the securement member 140, the elongate shaft 110, the release wire 120, and/or the vascular occlusion system 100, other orientations and/or positioning may be used. For example, in some embodiments, the joint 150 and/or the series of apertures may be oriented and/or positioned within or along a plane at an oblique angle to the longitudinal axis of the securement member 140, the elongate shaft 110, the release wire 120, and/or the vascular occlusion system 100. Other, for example non-planar, configurations are also possible. The proximal portion 142 of the securement member 140 is disposed proximal of the joint 150 and the distal portion 144 of the securement member 140 is disposed distal of the joint 150. As mentioned above, the proximal portion 142 of the securement member 140 may be releasably secured to and/or configured to disengage from the distal portion 144 of the securement member 140 at the joint 150 formed in the wall of the securement member 140.

In at least some embodiments, the frangible link may include a thinned and/or weakened feature, or series of features, formed in the wall of the securement member 140 that is more susceptible to fracture and/or separation than the remainder of the wall. In some embodiments, the frangible link may extend circumferentially about an entire circumference of the wall of the securement member 140. In some embodiments, the frangible link may extend partially and/or intermittently about the entire circumference of the wall of the securement member 140. The proximal portion 142 of the securement member 140 is disposed proximal of the frangible link and the distal portion 144 of the securement member 140 is disposed distal of the frangible link. As mentioned above, the proximal portion 142 of the securement member 140 may be releasably secured to and/or configured to disengage from the distal portion 144 of the securement member 140 at the frangible link formed in the wall of the securement member 140.

In some embodiments, the joint 150 of the securement member 140 may include both the perforation and the frangible link. For example, the perforation may be formed within the frangible link. In some embodiments, a portion of the circumference of the securement member 140 may include the perforation while a different portion of the circumference of the securement member 140 may include the frangible link. Other combinations and/or configuration are also contemplated.

In at least some embodiments, the securement member 140 may prevent axial translation of the release wire 120 relative to the elongate shaft 110 and/or the plurality of occlusive medical devices 130 prior to disengagement of the proximal portion 142 of the securement member 140 from the distal portion 144 of the securement member 140. Disengaging the proximal portion 142 of the securement member 140 from the distal portion 144 of the securement member 140 may permit the release wire 120 to axially translate relative to the distal portion 144 of the securement member 140, the elongate shaft 110, and/or the plurality of occlusive medical devices 130. In other words, the wall of the distal portion 144 of the securement member 140 may define a lumen, wherein the release wire 120 is slidably disposed within the lumen of the distal portion 144 of the securement member 140. Upon disengagement of the proximal portion 142 of the securement member 140 from the distal portion 144 of the securement member 140, as seen in FIGS. 4 and 5 for example, axial translation of the proximal portion 142 relative to the distal portion 144 of the securement member 140, the elongate shaft 110, and/or the plurality of occlusive medical devices 130 may translate the release wire 120 relative to the elongate shaft 110 and/or the distal portion 144 of the securement member 140 to release at least one of the plurality of occlusive medical devices 130 from the distal end 116 of the elongate shaft 110, as will be explained in more detail herein.

In some embodiments, and as illustrated in FIG. 3, the first attachment mechanism 170, the second attachment mechanism 180, and/or the third attachment mechanism 190 may be constructed in a substantially similar manner. Some suitable but non-limiting materials for the first attachment mechanism 170, the second attachment mechanism 180, and/or the third attachment mechanism 190, for example metallic materials, polymer materials, composite materials, etc., are described below.

The first attachment mechanism 170 may comprise the first part 172 having a first longitudinal lumen 174 configured to slidably receive the release wire 120 therein, and the second part 176 having a second longitudinal lumen 178 configured to slidably receive the release wire 120 therein. The first part 172 of the first attachment mechanism 170 may be fixedly attached to the distal end 116 of the elongate shaft 110 and the second part 176 of the first attachment mechanism 170 may be fixedly attached to the proximal end of the first occlusive medical device 132. The first part 172 of the first attachment mechanism 170 and the second part 176 of the first attachment mechanism 170 may be configured to interlock with each other such that relative axial translation between the first part 172 of the first attachment mechanism 170 and the second part 176 of the first attachment mechanism 170 is prevented when a face of the first part 172 of the first attachment mechanism 170 abuts and/or engages a face of the second part 176 of the first attachment mechanism 170 and the first longitudinal lumen 174 of the first part 172 of the first attachment mechanism 170 is aligned coaxially with the second longitudinal lumen 178 of the second part 176 of the first attachment mechanism 170. Additionally, in some embodiments, the first part 172 of the first attachment mechanism 170 and the second part 176 of the first attachment mechanism 170 may be configured to interlock with each other such that relative lateral translation between the first part 172 of the first attachment mechanism 170 and the second part 176 of the first attachment mechanism 170 is prevented when a face of the first part 172 of the first attachment mechanism 170 abuts and/or engages a face of the second part 176 of the first attachment mechanism 170, the first longitudinal lumen 174 of the first part 172 of the first attachment mechanism 170 is aligned coaxially with the second longitudinal lumen 178 of the second part 176 of the first attachment mechanism 170, and the release wire 120 is slidably engaged with the first longitudinal lumen 174 of the first part 172 of the first attachment mechanism 170 and the second longitudinal lumen 178 of the second part 176 of the first attachment mechanism 170.

The second attachment mechanism 180 may comprise the third part 182 having a third longitudinal lumen 184 configured to slidably receive the release wire 120 therein, and the fourth part 186 having a fourth longitudinal lumen 188 configured to slidably receive the release wire 120 therein. The third part 182 of the second attachment mechanism 180 may be fixedly attached to the distal end of the first occlusive medical device 132 and the fourth part 186 of the second attachment mechanism 180 may be fixedly attached to the proximal end of the second occlusive medical device 134. The third part 182 of the second attachment mechanism 180 and the fourth part 186 of the second attachment mechanism 180 may be configured to interlock with each other such that relative axial translation between the third part 182 of the second attachment mechanism 180 and the fourth part 186 of the second attachment mechanism 180 is prevented when a face of the third part 182 of the second attachment mechanism 180 abuts and/or engages a face of the fourth part 186 of the second attachment mechanism 180 and the third longitudinal lumen 184 of the third part 182 of the second attachment mechanism 180 is aligned coaxially with the fourth longitudinal lumen 188 of the fourth part 186 of the second attachment mechanism 180. Additionally, in some embodiments, the third part 182 of the second attachment mechanism 180 and the fourth part 186 of the second attachment mechanism 180 may be configured to interlock with each other such that relative lateral translation between the third part 182 of the second attachment mechanism 180 and the fourth part 186 of the second attachment mechanism 180 is prevented when a face of the third part 182 of the second attachment mechanism 180 abuts and/or engages a face of the fourth part 186 of the second attachment mechanism 180, the third longitudinal lumen 184 of the third part 182 of the second attachment mechanism 180 is aligned coaxially with the fourth longitudinal lumen 188 of the fourth part 186 of the second attachment mechanism 180, and the release wire 120 is slidably engaged with the third longitudinal lumen 184 of the third part 182 of the second attachment mechanism 180 and the fourth longitudinal lumen 188 of the fourth part 186 of the second attachment mechanism 180.

The third attachment mechanism 190 may comprise the fifth part 192 having a fifth longitudinal lumen 194 configured to slidably receive the release wire 120 therein, and the sixth part 196 having a sixth longitudinal lumen 198 configured to slidably receive the release wire 120 therein. The fifth part 192 of the third attachment mechanism 190 may be fixedly attached to the distal end of the second occlusive medical device 134 and the sixth part 196 of the third attachment mechanism 190 may be fixedly attached to the proximal end of the third occlusive medical device 136. The fifth part 192 of the third attachment mechanism 190 and the sixth part 196 of the third attachment mechanism 190 may be configured to interlock with each other such that relative axial translation between the fifth part 192 of the third attachment mechanism 190 and the sixth part 196 of the third attachment mechanism 190 is prevented when a face of the fifth part 192 of the third attachment mechanism 190 abuts and/or engages a face of the sixth part 196 of the third attachment mechanism 190 and the fifth longitudinal lumen 194 of the fifth part 192 of the third attachment mechanism 190 is aligned coaxially with the sixth longitudinal lumen 198 of the sixth part 196 of the third attachment mechanism 190. Additionally, in some embodiments, the fifth part 192 of the third attachment mechanism 190 and the sixth part 196 of the third attachment mechanism 190 may be configured to interlock with each other such that relative lateral translation between the fifth part 192 of the third attachment mechanism 190 and the sixth part 196 of the third attachment mechanism 190 is prevented when a face of the fifth part 192 of the third attachment mechanism 190 abuts and/or engages a face of the sixth part 196 of the third attachment mechanism 190, the fifth longitudinal lumen 194 of the fifth part 192 of the third attachment mechanism 190 is aligned coaxially with the sixth longitudinal lumen 198 of the sixth part 196 of the third attachment mechanism 190, and the release wire 120 is slidably engaged with the fifth longitudinal lumen 194 of the fifth part 192 of the third attachment mechanism 190 and the sixth longitudinal lumen 198 of the sixth part 196 of the third attachment mechanism 190.

FIGS. 4 and 5 generally illustrate at least one of the plurality of occlusive medical devices 130 being released from the distal end 116 of the elongate shaft 110, such as at a treatment site, for example. In use, the microcatheter 200 of the vascular occlusion system 100 may be inserted into a patient's anatomy and/or vasculature and a distal end guided and/or advanced to a location adjacent a treatment site. The plurality of occlusive medical devices 130 disposed at the distal end 116 of the elongate shaft 110 may be inserted into a proximal end of the lumen 202 disposed within the microcatheter 200 and advanced through the microcatheter 200 to the treatment site. In some embodiments, the plurality of occlusive medical devices 130 may be disposed within the lumen 202 of the microcatheter 200 proximate to the distal end 116 of the elongate shaft 110. In some embodiments, the plurality of occlusive medical devices 130 may be disposed within the lumen 202 of the microcatheter 200 proximate to the distal end 116 of the elongate shaft 110 prior to use and/or prior to inserting the microcatheter 200 into the patient's anatomy and/or vasculature.

Deployment and/or release of at least one of the plurality of occlusive medical devices 130 may be performed selectively depending upon the type of occlusive medical devices and/or the desired treatment process or method. It has been found that the exact amount of occlusive medical device (e.g., embolic coil, etc.) needed to properly occlude a vasculature (e.g., an artery, vein, etc.) or treatment site may not be known until the treatment procedure is underway, due to variations in the size of the vasculature, plaque build-up within the vasculature, distention of the vasculature, etc. If an insufficient amount of occlusive medical device is provided to the treatment site initially, additional occlusive medical device(s) may need to be prepared and inserted into the patient's vasculature via additional vascular occlusion system(s), thus extending the treatment procedure and any attendant risks to the patient. In order to reduce the treatment procedure duration and to provide more flexibility in deploying and/or releasing the required amount of occlusive medical device(s), the vascular occlusion system 100 has been developed which includes a plurality of occlusive medical devices 130 releasably connected to the distal end 116 of the elongate shaft 110. Using the vascular occlusion system 100 described herein, an operator may selectively deploy one or more of the plurality of occlusive medical devices 130 based upon the individual procedure requirements, while requiring only a single vascular occlusion system, thus reducing the treatment procedure duration and the overall cost of the treatment procedure (due to fewer vascular occlusion systems, along with the attendant packaging, preparation requirements, etc., being used).

When ready to deploy at least one of the plurality of occlusive medical devices 130 at the treatment site, the elongate shaft 110 may be advanced and/or translated distally relative to the microcatheter 200 until at least one of the plurality of occlusive medical devices 130 is exposed and/or disposed distal of the microcatheter 200. The elongate shaft 110 may have sufficient length that the proximal end 114 of the elongate shaft 110 and/or the securement member 140 remains proximal of (e.g., extends proximally from) the microcatheter 200 when the plurality of occlusive medical devices 130 is disposed distal of the microcatheter 200. In use, the elongate shaft 110 and the microcatheter 200 may have sufficient length to reach from the treatment site to a position outside of the patient where the vascular occlusion system 100 may be manipulated by an operator (e.g., clinician, physician, user, etc.). The operator of the vascular occlusion system 100 may then place a first hand on the distal portion 144 of the securement member 140 and a second hand on the proximal portion 142 of the securement member 140. The proximal portion 142 of the securement member 140 may be configured to disengage from the distal portion 144 of the securement member 140 at a location proximal of a proximal end of the microcatheter 200 when the plurality of occlusive medical devices 130 is disposed distal of the microcatheter 200. In at least some embodiments, the proximal portion 142 of the securement member 140 may be disengaged from the distal portion 144 of the securement member 140 by bending, twisting, and/or pulling the proximal portion 142 of the securement member 140 relative to the distal portion 144 of the securement member 140. In some embodiments, disengaging the proximal portion 142 of the securement member 140 from the distal portion 144 of the securement member 140 may include moving the proximal portion 142 of the securement member 140 relative to the distal portion 144 of the securement member 140 to separate the proximal portion 142 of the securement member 140 from the distal portion 144 of the securement member 140. In some embodiments, disengaging the proximal portion 142 of the securement member 140 from the distal portion 144 of the securement member 140 may include using an external device (e.g., a torque device, an external handle, etc.) to move the proximal portion 142 of the securement member 140 relative to the distal portion 144 of the securement member 140.

When the proximal portion 142 of the securement member 140 is disengaged and/or separated from the distal portion 144 of the securement member 140, as seen in FIG. 4, the release wire 120 is translated in a proximal direction relative to the elongate shaft 110 and/or the plurality of occlusive medical devices 130 to release the sixth part 196 of the third attachment mechanism 190 and/or the third occlusive medical device 136 from the fifth part 192 of the third attachment mechanism 190 and/or the elongate shaft 110 (e.g., to release the third occlusive medical device 136 from the distal end 116 of the elongate shaft 110). When the release wire 120 is translated and/or withdrawn proximally relative to the elongate shaft 110 and/or the third occlusive medical device 136, the third occlusive medical device 136 is released from the distal end 116 of the elongate shaft 110 and shifts from the first shape and/or the elongated delivery configuration to the second shape and/or the deployed configuration.

The release wire 120 includes one or more indicators 128 disposed proximate the proximal end of the release wire 120 configured to show how much of the release wire 120 has been withdrawn and/or configured to communicate to a user of the vascular occlusion system 100 how much or how many of the plurality of occlusive medical devices 130 has been released, as seen in FIGS. 4 and 5 for example. The one or more indicators 128 may include lines, detents, colors, notches, or other suitable indicators. The one or more indicators 128, if visible, may be seen between the proximal portion 142 of the securement member 140 and the distal portion 144 of the securement member 140 after the proximal portion 142 of the securement member 140 has been disengaged from the distal portion 144 of the securement member 140 at the joint 150, as described above.

After releasing one of the plurality of occlusive medical devices 130 (e.g., the third occlusive medical device 136) at the treatment site, the vascular occlusion system 100 and/or the elongate shaft 110 may be advanced distally, if necessary, to position the distal end of one of the plurality of occlusive medical devices 130 (e.g., the second occlusive medical device 134 and/or the first occlusive medical device 132) still connected to the distal end 116 of the elongate shaft 110 adjacent the released occlusive medical device (e.g., the third occlusive medical device 136). The release wire 120 may then be further translated proximally relative to the elongate shaft 110 and/or the plurality of occlusive medical devices 130 to release the positioned occlusive medical device (e.g., the second occlusive medical device 134 and/or the first occlusive medical device 132) from the distal end 116 of the elongate shaft 110, as seen in FIG. 5 for example. When the release wire 120 is translated and/or withdrawn proximally relative to the elongate shaft 110 and/or the second occlusive medical device 134, the second occlusive medical device 134 is released from the distal end 116 of the elongate shaft 110 and shifts from the first shape and/or the elongated delivery configuration to the second shape and/or the deployed configuration.

After releasing two of the plurality of occlusive medical devices 130 (e.g., the second occlusive medical device 134 and the third occlusive medical device 136) at the treatment site, the vascular occlusion system 100 and/or the elongate shaft 110 may be advanced distally, if necessary, to position the distal end of one of the plurality of occlusive medical devices 130 (e.g., the first occlusive medical device 132) still connected to the distal end 116 of the elongate shaft 110 adjacent the released occlusive medical device(s) (e.g., the third occlusive medical device 136 and/or the second occlusive medical device 134). The release wire 120 may then be further translated proximally relative to the elongate shaft 110 and/or the plurality of occlusive medical devices 130 to release the positioned occlusive medical device (e.g., the first occlusive medical device 132) from the distal end 116 of the elongate shaft 110. When the release wire 120 is translated and/or withdrawn proximally relative to the elongate shaft 110 and/or the first occlusive medical device 132, the first occlusive medical device 132 is released from the distal end 116 of the elongate shaft 110 and shifts from the first shape and/or the elongated delivery configuration to the second shape and/or the deployed configuration.

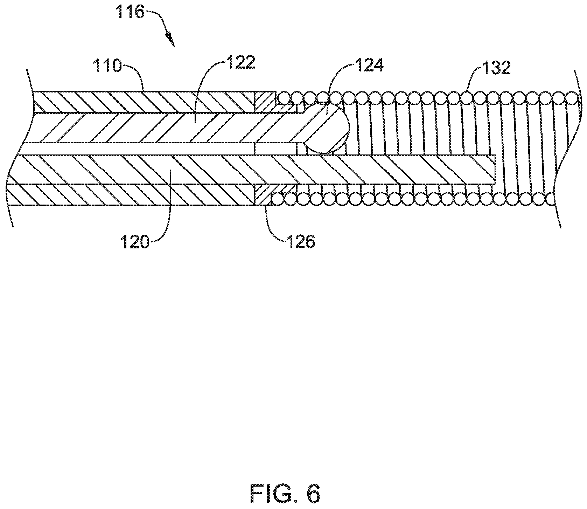

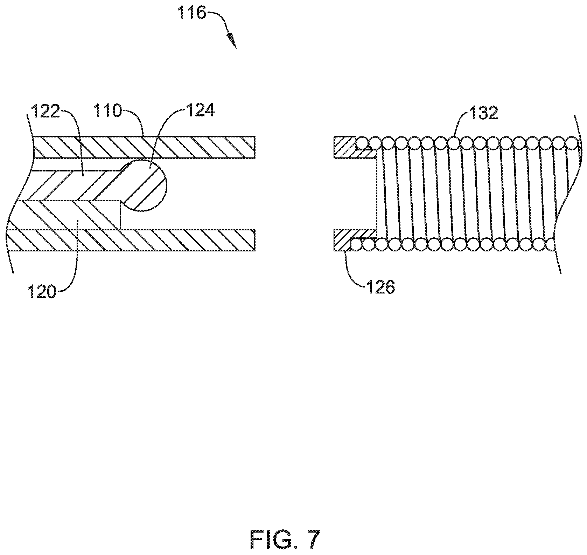

FIGS. 6 and 7 illustrate an alternative attachment and/or release mechanism for use with the plurality of occlusive medical devices 130 described herein. While illustrated with respect to the first occlusive medical device 132 and the distal end 116 of the elongate shaft 110, the illustrated alternative attachment and/or release mechanism may be used with any and/or each of the plurality of occlusive medical devices 130. In embodiments using the alternative attachment and/or release mechanism, the vascular occlusion system 100 may further comprise a second release wire 122 slidably disposed within the lumen 112 of the elongate shaft 110 and at least a portion of each of the plurality of occlusive medical devices 130. The second release wire 122 may include a ball tip 124 fixedly attached to and/or disposed at a distal end of the second release wire 122, wherein the ball tip 124 has an outer diameter greater than an outer diameter of the second release wire 122. The release wire 120 extends distally of the ball tip 124 of the second release wire 122 when the release wire 120 and the second release wire 122 are slidably disposed within at least a portion of each of the plurality of occlusive medical devices 130.

The vascular occlusion system 100 may further comprise a securing member 126 fixedly attached to a proximal end of each of the plurality of occlusive medical devices 130 (e.g., the first occlusive medical device 132, the second occlusive medical device 134, and/or the third occlusive medical device 136). The release wire 120 and the second release wire 122 may be configured to releasably connect, attach, and/or secure the plurality of occlusive medical devices 130 to the distal end 116 of the elongate shaft 110 when the release wire 120 and the second release wire 122 are disposed within at least a portion of each of the plurality of occlusive medical devices 130 and/or when both the release wire 120 and the second release wire 122 extend through the securing member 126 simultaneously. When both the release wire 120 and the second release wire 122 extend through the securing member 126 simultaneously, as shown in FIG. 6 for example, the second release wire 122 is prevented from being withdrawn through the securing member 126 due to interference between the ball tip 124 and the securing member 126.

Proximal withdrawal of the release wire 120 and then the second release wire 122 through the securing member 126 and/or relative to the elongate shaft 110 releases one of the plurality of occlusive medical devices 130 (e.g., the first occlusive medical device 132, the second occlusive medical device 134, and/or the third occlusive medical device 136) from the distal end 116 of the elongate shaft 110. For example, by withdrawing the release wire 120 through the securing member 126, the second release wire 122 is permitted to deflect radially inward as it contacts and/or passes through the securing member 126 instead of interfering with the securing member 126 when the ball tip 124 contacts the securing member 126, wherein the ball tip 124 is prevented from deflecting by the release wire 120. Thus, proximal withdrawal of the release wire 120 and then the second release wire 122 through the securing member 126 and/or relative to the elongate shaft 110 releases one of the plurality of occlusive medical devices 130 (e.g., the first occlusive medical device 132, as shown in FIG. 7 for example) from the distal end 116 of the elongate shaft 110. As mentioned above, the same process may apply to each of the plurality of occlusive medical devices 130.

In some embodiments, the release wire 120 and the second release wire 122 may extend completely through at least one of the plurality of occlusive medical devices 130. In some embodiments, the release wire 120 and the second release wire 122 may extend completely through the first occlusive medical device 132 and the second occlusive medical device 134, and into at least a portion of the third occlusive medical device 136. In some embodiments, proximal withdrawal of the release wire 120 and then the second release wire 122 relative to the elongate shaft 110 and/or the plurality of occlusive medical devices 130 sequentially releases each of the plurality of occlusive medical devices 130 from a distalmost occlusive medical device (e.g., the third occlusive medical device 136) to a proximalmost occlusive medical device (e.g., the first occlusive medical device 132). In some embodiments, the second release wire 122 may be alternately and/or interchangeably referred to as a pull wire, an actuation wire, and/or a locking wire. The second release wire 122 may generally be a solid wire or shaft, but may also be tubular in some embodiments. Some suitable but non-limiting materials for and the second release wire 122, for example metallic materials, polymer materials, composite materials, etc., are described below.

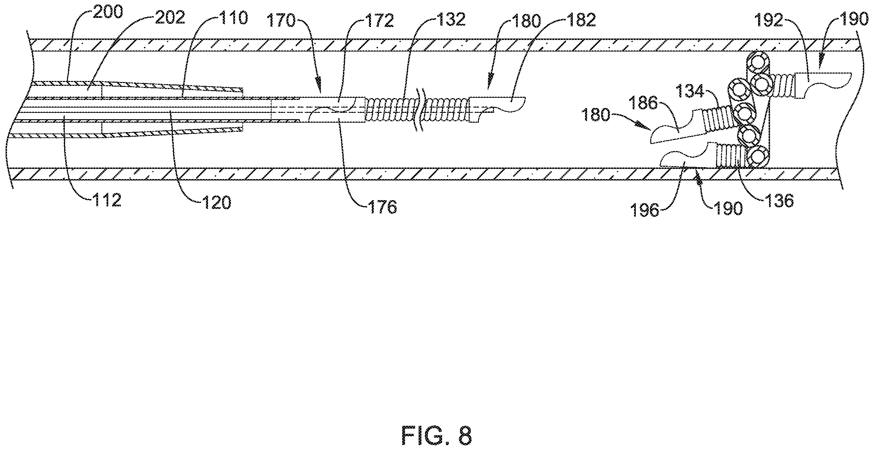

The vascular occlusion system 100 may be used in a method of embolizing a vasculature (e.g., an artery, a vein, etc.), aspects of which are illustrated in FIG. 8. The method may comprise advancing the vascular occlusion system 100 to the treatment within the vasculature (e.g., an artery, a vein, etc.). As discussed above, the vascular occlusion system 100 may include the plurality of occlusive medical devices 130 releasably connected to the distal end 116 of the elongate shaft 110, and a release wire 120 slidably disposed within the lumen 112 of the elongate shaft 110 and at least a portion of each of the plurality of occlusive medical devices 130.

The method may further comprise withdrawing the release wire 120 proximally relative to the elongate shaft 110 and/or the plurality of occlusive medical devices 130 to release one of the plurality of occlusive medical devices 130 from the distal end 116 of the elongate shaft 110 at the treatment site. For example, withdrawing the release wire 120 proximally relative to the elongate shaft 110 and/or the plurality of occlusive medical devices 130 may release the third occlusive medical device 136 from the distal end 116 of the elongate shaft 110 at the treatment site by releasing the sixth part 196 of the third attachment mechanism 190 from the fifth part 192 of the third attachment mechanism 190. In at least some embodiments, the third occlusive medical device 136 (e.g., the one of the plurality of occlusive medical devices 130) may be configured to shift from the first shape and/or the elongated delivery configuration to the second shape and/or the deployed configuration within and/or against a wall of the vasculature (e.g., the artery, the vein, etc.) and/or the treatment site. In some embodiments, the third occlusive medical device 136 (e.g., the one of the plurality of occlusive medical devices 130) may be configured to assume a substantially helical configuration and/or shape within and/or against the wall of the vasculature (e.g., the artery, the vein, etc.) and/or the treatment site. Other configurations and/or shapes are also contemplated. The third occlusive medical device 136 (e.g., the one of the plurality of occlusive medical devices 130) has a stiffness greater than any other of the plurality of occlusive medical devices 130 to form a solid "base" or "anchor" within the vasculature (e.g., the artery, the vein, etc.) and/or the treatment site, thereby preventing migration of the deployed plurality of occlusive medical devices 130.

The method may further comprise positioning the distal end of one of the plurality of occlusive medical devices 130 (e.g., the second occlusive medical device 134, the first occlusive medical device 132, etc.) still connected to the distal end 116 of the elongate shaft 110 adjacent the released occlusive medical device (e.g., the third occlusive medical device 136, the one of the plurality of occlusive medical devices 130, etc.). For example, the method may comprise positioning the distal end of the second occlusive medical device 134 and/or the fifth part 192 of the third attachment mechanism 190 adjacent the third occlusive medical device 136, which was previously deployed within the vasculature (e.g., the artery, the vein, etc.) and/or the treatment site.

The method may further comprise withdrawing the release wire 120 proximally relative to the elongate shaft 110 and/or the plurality of occlusive medical devices 130 to release the positioned occlusive medical device (e.g., the second occlusive medical device 134) from the distal end 116 of the elongate shaft 110 at the treatment site. For example, withdrawing the release wire 120 proximally relative to the elongate shaft 110 and/or the plurality of occlusive medical devices 130 may release the second occlusive medical device 134 from the distal end 116 of the elongate shaft 110 at the treatment site by releasing the fourth part 186 of the second attachment mechanism 180 from the third part 182 of the second attachment mechanism 180. In at least some embodiments, the second occlusive medical device 134 (e.g., the positioned occlusive medical device) may be configured to shift from the first shape and/or the elongated delivery configuration to the second shape and/or the deployed configuration within the vasculature (e.g., the artery, the vein, etc.) and/or the treatment site. In some embodiments, the second occlusive medical device 134 (e.g., the positioned occlusive medical device) may be configured to assume a configuration and/or a second shape within the vasculature (e.g., the artery, the vein, etc.) and/or the treatment site that is different from the third occlusive medical device 136. In some embodiments, the second occlusive medical device 134 (e.g., the positioned occlusive medical device) may assume a substantially randomized and/or irregular second shape. Other configurations and/or shapes are also contemplated. The second occlusive medical device 134 (e.g., the positioned occlusive medical device) has a stiffness less than the third occlusive medical device 136 (e.g., the one of the plurality of occlusive medical devices 130), thereby permitting easier "packing" of the second occlusive medical device 134 against, within, and/or around the third occlusive medical device 136 to fill and/or occlude the vasculature (e.g., the artery, the vein, etc.) and/or the treatment site.

After releasing two of the plurality of occlusive medical devices 130 (e.g., the second occlusive medical device 134 and the third occlusive medical device 136) at the treatment site, the method may further comprise positioning the distal end of one of the plurality of occlusive medical devices 130 (e.g., the first occlusive medical device 132) still connected to the distal end 116 of the elongate shaft 110 adjacent the released occlusive medical device (e.g., the second occlusive medical device 134 and/or the third occlusive medical device 136, the one of the plurality of occlusive medical devices 130, etc.). For example, the method may comprise positioning the distal end of the first occlusive medical device 132 and/or the third part 182 of the second attachment mechanism 180 adjacent the third occlusive medical device 136 and/or the second occlusive medical device 134, which was previously deployed within the vasculature (e.g., the artery, the vein, etc.) and/or the treatment site.

The method may further comprise withdrawing the release wire 120 proximally relative to the elongate shaft 110 and/or the plurality of occlusive medical devices 130 to release the positioned occlusive medical device (e.g., the first occlusive medical device 132) from the distal end 116 of the elongate shaft 110 at the treatment site. For example, withdrawing the release wire 120 proximally relative to the elongate shaft 110 and/or the plurality of occlusive medical devices 130 may release the first occlusive medical device 132 from the distal end 116 of the elongate shaft 110 at the treatment site by releasing the second part 176 of the first attachment mechanism 170 from the first part 172 of the first attachment mechanism 170. In at least some embodiments, the first occlusive medical device 132 (e.g., the positioned occlusive medical device) may be configured to shift from the first shape and/or the elongated delivery configuration to the second shape and/or the deployed configuration within the vasculature (e.g., the artery, the vein, etc.) and/or the treatment site. In some embodiments, the first occlusive medical device 132 (e.g., the positioned occlusive medical device) may be configured to assume a configuration and/or a second shape within the vasculature (e.g., the artery, the vein, etc.) and/or the treatment site that is different from the third occlusive medical device 136 and/or the second occlusive medical device 134. In some embodiments, the first occlusive medical device 132 (e.g., the positioned occlusive medical device) may assume a substantially randomized and/or irregular second shape. Other configurations and/or shapes are also contemplated. The first occlusive medical device 132 (e.g., the positioned occlusive medical device) has a stiffness less than the third occlusive medical device 136 and possibly also the second occlusive medical device 134 (e.g., the one of the plurality of occlusive medical devices 130), thereby permitting easier "packing" of the first occlusive medical device 132 against, within, and/or around the third occlusive medical device 136 and/or the second occlusive medical device 134 to fill and/or occlude the vasculature (e.g., the artery, the vein, etc.) and/or the treatment site.

In some embodiments, proximal withdrawal of the release wire 120 relative to the elongate shaft 110 and/or the plurality of occlusive medical devices 130 sequentially releases each of the plurality of occlusive medical devices 130 from a distalmost occlusive medical device (e.g., the third occlusive medical device 136) to a proximalmost occlusive medical device (e.g., the first occlusive medical device 132).

The materials that can be used for the various components of the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc. (and/or other systems disclosed herein) and the various elements thereof disclosed herein may include those commonly associated with medical devices. For simplicity purposes, the following discussion makes reference to the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc. However, this is not intended to limit the devices and methods described herein, as the discussion may be applied to other elements, members, components, or devices disclosed herein, such as, but not limited to, the first occlusive medical device 132, the second occlusive medical device 134, the third occlusive medical device 136, the first part 172, the second part 176, the third part 182, the fourth part 186, the fifth part 192, the sixth part 196, etc. and/or elements or components thereof.

In some embodiments, the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc., and/or components thereof (such as, but not limited to, the first occlusive medical device 132, the second occlusive medical device 134, the third occlusive medical device 136, the first part 172, the second part 176, the third part 182, the fourth part 186, the fifth part 192, the sixth part 196, etc.), may be made from a metal, metal alloy, polymer (some examples of which are disclosed below), a metal-polymer composite, ceramics, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; platinum; palladium; gold; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANUIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc., and/or components thereof, may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc. to achieve the same result.

In some embodiments, a degree of Magnetic Resonance Imaging (MRI) compatibility is imparted into the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc. For example, the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc., and/or components or portions thereof, may be made of a material that does not substantially distort the image and create substantial artifacts (e.g., gaps in the image). Certain ferromagnetic materials, for example, may not be suitable because they may create artifacts in an MRI image. The vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc., or portions thereof, may also be made from a material that the MRI machine can image. Some materials that exhibit these characteristics include, for example, tungsten, cobalt-chromium-molybdenum alloys (e.g., UNS: R44003 such as ELGILOY^{®}, PHYNOX^{®}, and the like), nickel-cobalt-chromium-molybdenum alloys (e.g., UNS: R44035 such as MP35-N^{®} and the like), nitinol, and the like, and others.

In some embodiments, the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc., and/or portions thereof, may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-6-isobutylene-6-styrene) (for example, SIBS and/or SIBS 50A), polycarbonates, ionomers, polyurethane silicone copolymers (for example, ElastEon^{®} from Aortech Biomaterials or ChronoSil^{®} from AdvanSource Biomaterials), biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc. and/or other elements disclosed herein may include a fabric material disposed over or within the structure. The fabric material may be composed of a biocompatible material, such a polymeric material or biomaterial, adapted to promote tissue ingrowth. In some embodiments, the fabric material may include a bioabsorbable material. Some examples of suitable fabric materials include, but are not limited to, polyethylene glycol (PEG), nylon, polytetrafluoroethylene (PTFE, ePTFE), a polyolefinic material such as a polyethylene, a polypropylene, polyester, polyurethane, and/or blends or combinations thereof.

In some embodiments, the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc. may include and/or be formed from a textile material. Some examples of suitable textile materials may include synthetic yarns that may be flat, shaped, twisted, textured, pre-shrunk or un-shrunk. Synthetic biocompatible yarns suitable for use in the present invention include, but are not limited to, polyesters, including polyethylene terephthalate (PET) polyesters, polypropylenes, polyethylenes, polyurethanes, polyolefins, polyvinyls, polymethylacetates, polyamides, naphthalene dicarboxylene derivatives, natural silk, and polytetrafluoroethylenes. Moreover, at least one of the synthetic yarns may be a metallic yarn or a glass or ceramic yarn or fiber. Useful metallic yarns include those yarns made from or containing stainless steel, platinum, gold, titanium, tantalum or a Ni-Co-Cr-based alloy. The yarns may further include carbon, glass or ceramic fibers. Desirably, the yarns are made from thermoplastic materials including, but not limited to, polyesters, polypropylenes, polyethylenes, polyurethanes, polynaphthalenes, polytetrafluoroethylenes, and the like. The yarns may be of the multifilament, monofilament, or spun-types. The type and denier of the yarn chosen may be selected in a manner which forms a biocompatible and implantable prosthesis and, more particularly, a vascular structure having desirable properties.

In some embodiments, the vascular occlusion system 100, the elongate shaft 110, the release wire 120, the second release wire 122, the securing member 126, the plurality of occlusive medical devices 130, the securement member 140, the first attachment mechanism 170, the second attachment mechanism 180, the third attachment mechanism 190, the microcatheter 200, etc. may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vascoactive mechanisms.

It should be understood that this disclosure is, in many respects, only illustrative. This may include, to the extent that it is appropriate, the use of any of the features of one example embodiment being used in other embodiments. The invention's scope is, of course, defined in the language in which the appended claims are expressed.

## Claims

1. A vascular occlusion system (100), comprising:
a microcatheter (200) configured to navigate a vasculature;
an elongate shaft (110) slidably disposed within a lumen (202) of the microcatheter (200), the elongate shaft (110) having a lumen (112) extending from a proximal end (114) of the elongate shaft (110) to a distal end (116) of the elongate shaft (110);
a plurality of occlusive medical devices (130) releasably connected to the elongate shaft (110),
wherein at least one of the plurality of occlusive medical devices (130) has a different stiffness than a different one of the plurality of occlusive medical devices (130),
wherein a distalmost one of the plurality of the occlusive medical devices (130) has a stiffness greater than any other of the plurality of occlusive medical devices; and
a release wire (120) slidably disposed within the elongate shaft (110) and at least a portion of each of the plurality of occlusive medical devices (130);
wherein the release wire (120) secures each of the plurality of occlusive medical devices (130) to the distal end (116) of the elongate shaft (110) when the release wire (120) is disposed within at least a portion of each of the plurality of occlusive medical devices (130),
wherein the release wire (120) includes one or more indicators (128) disposed proximate the proximal end of the release wire (120) configured to show how much of the release wire (120) has been withdrawn and/or configured to communicate to a user of the vascular occlusion system (100) how much or how many of the plurality of occlusive medical devices (130) has been released.

2. The vascular occlusion system of claim 1, wherein the plurality of occlusive medical devices (130) includes a first occlusive medical device (132) having a proximal end disposed adjacent the distal end (116) of the elongate shaft (110) and a second occlusive medical device (134) having a proximal end disposed adjacent a distal end of the first occlusive medical device (132).

3. The vascular occlusion system of claim 2, further comprising a first attachment mechanism (170) disposed between the distal end (116) of the elongate shaft (110) and the proximal end of the first occlusive medical device (132);
wherein the first attachment mechanism (170) comprises:
a first part (172) having a first longitudinal lumen (174) configured to slidably receive the release wire (120), and a second part (176) having a second longitudinal lumen (178) configured to slidably receive the release wire (120);
wherein the first part (172) is fixedly attached to the distal end (116) of the elongate shaft (110) and the second part (176) is fixedly attached to the proximal end of the first occlusive medical device (132);
wherein the first part (172) and the second part (176) are configured to interlock with each other such that relative axial translation between the first part (172) and the second part (176) is prevented when the first part (172) abuts the second part (176) and the first longitudinal lumen (174) is aligned coaxially with the second longitudinal lumen (178).

4. The vascular occlusion system of claim 3, wherein the first part (172) and the second part (176) are configured to interlock with each other such that relative lateral translation between the first part (172) and the second part (176) is prevented when the first part (172) abuts the second part (176), the first longitudinal lumen (174) is aligned coaxially with the second longitudinal lumen (178), and the release wire (120) is slidably engaged with the first longitudinal lumen (174) and the second longitudinal lumen (178).

5. The vascular occlusion system of claim 2, further comprising a second attachment mechanism (180) disposed between the distal end of the first occlusive medical device (132) and the proximal end of the second occlusive medical device (134);
wherein the second attachment mechanism (180) comprises:
a third part (182) having a third longitudinal lumen (184) configured to slidably receive the release wire (120), and a fourth part (186) having a fourth longitudinal lumen (188) configured to slidably receive the release wire (120);
wherein the third part (182) is fixedly attached to the distal end of the first occlusive medical device (132) and the fourth part (186) is fixedly attached to the proximal end of the second occlusive medical device (134);
wherein the third part (182) and the fourth part (186) are configured to interlock with each other such that relative axial translation between the third part (182) and the fourth part (186) is prevented when the third part (182) abuts the fourth part (186) and the third longitudinal lumen (184) is aligned coaxially with the fourth longitudinal lumen (188).

6. The vascular occlusion system of claim 5, wherein the third part (182) and the fourth part (186) are configured to interlock with each other such that relative lateral translation between the third part (182) and the fourth part (186) is prevented when the third part (182) abuts the fourth part (186), the third longitudinal lumen (184) is aligned coaxially with the fourth longitudinal lumen (188), and the release wire (120) is slidably engaged with the third longitudinal lumen (184) and the fourth longitudinal lumen (188).

7. The vascular occlusion system of claim 2, wherein the plurality of occlusive medical devices (130) includes a third occlusive medical device (136) having a proximal end disposed adjacent a distal end of the second occlusive medical device (134).

8. The vascular occlusion system of claim 7, further comprising a third attachment mechanism (190) disposed between the distal end of the second occlusive medical device (134) and the proximal end of the third occlusive medical device (136);
wherein the third attachment mechanism (190) comprises:
a fifth part (192) having a fifth longitudinal lumen (194) configured to slidably receive the release wire (120), and a sixth part (196) having a sixth longitudinal lumen (198) configured to slidably receive the release wire (120);
wherein the fifth part (192) is fixedly attached to the distal end of the second occlusive medical device (134) and the sixth part (196) is fixedly attached to the proximal end of the third occlusive medical device (136);
wherein the fifth part (192) and the sixth part (196) are configured to interlock with each other such that relative axial translation between the fifth part (192) and the sixth part (196) is prevented when the fifth part (192) abuts the sixth part (196) and the fifth longitudinal lumen (194) is aligned coaxially with the sixth longitudinal lumen (198).

9. The vascular occlusion system of claim 8, wherein the fifth part (192) and the sixth part (196) are configured to interlock with each other such that relative lateral translation between the fifth part (192) and the sixth part (196) is prevented when the fifth part (192) abuts the sixth part (196), the fifth longitudinal lumen (194) is aligned coaxially with the sixth longitudinal lumen (198), and the release wire (120) is slidably engaged with the fifth longitudinal lumen (194) and the sixth longitudinal lumen (198).

10. The vascular occlusion system of any of claims 1-9, wherein at least one of the plurality of occlusive medical devices (130) is a coiled member configured to assume a first shape when connected to the elongate shaft (110) and a second shape when disconnected from the elongate shaft (110).

11. The vascular occlusion system of any of claims 1-10, wherein at least one of the plurality of occlusive medical devices (130) has a different length than a different one of the plurality of occlusive medical devices (130).

12. The vascular occlusion system of claim 1, further comprising a second release wire (122) slidably disposed within the elongate shaft (110) and at least a portion of each of the plurality of occlusive medical devices (130), wherein the second release wire (122) includes a ball tip (124).

13. The vascular occlusion system of claim 12, further comprising a securing member (126) fixedly attached to a proximal end of each of the plurality of occlusive medical devices (130), wherein when the release wire (120) and the second release wire (122) both extend through the securing member (126) simultaneously, the second release wire (122) is prevented from being withdrawn through the securing member (126).

## Patentansprüche

1. Gefäßverschlusssystem (100), aufweisend:
einen Mikrokatheter (200), der zum Navigieren durch ein Gefäßsystem konfiguriert ist;
einen länglichen Schaft (110), der verschiebbar in einem Lumen (202) des Mikrokatheters (200) angeordnet ist, wobei der längliche Schaft (110) ein Lumen (112) hat, das sich von einem proximalen Ende (114) des länglichen Schafts (110) zu einem distalen Ende (116) des länglichen Schafts (110) erstreckt;
mehrere medizinische Verschlussvorrichtungen (130), die lösbar mit dem länglichen Schaft (110) verbunden sind,
wobei mindestens eine der mehreren medizinischen Verschlussvorrichtungen (130) eine andere Steifigkeit als eine andere der mehreren medizinischen Verschlussvorrichtungen (130) aufweist,
wobei eine am weitesten distal gelegene eine der mehreren medizinischen Verschlussvorrichtungen (130) eine größere Steifigkeit aufweist als jede andere der mehreren medizinischen Verschlussvorrichtungen; und
einen Freigabedraht (120), der verschiebbar innerhalb des länglichen Schafts (110) und mindestens eines Teils jeder der mehreren medizinischen Verschlussvorrichtungen (130) angeordnet ist;
wobei der Freigabedraht (120) jede der mehreren medizinischen Verschlussvorrichtungen (130) an dem distalen Ende (116) des länglichen Schafts (110) befestigt, wenn der Freigabedraht (120) innerhalb mindestens eines Teils jeder der mehreren medizinischen Verschlussvorrichtungen (130) angeordnet ist,
wobei der Freigabedraht (120) einen oder mehrere Indikatoren (128) aufweist, die in der Nähe des proximalen Endes des Freigabedrahts (120) angeordnet und so konfiguriert sind, dass sie zeigen, wie viel des Freigabedrahts (120) zurückgezogen wurde, und/oder so konfiguriert sind, dass sie einem Benutzer des Gefäßverschlusssystems (100) mitteilen, wie viel oder wie viele der mehreren medizinischen Verschlussvorrichtungen (130) freigegeben wurde oder wurden.

2. Gefäßverschlusssystem nach Anspruch 1, wobei die mehreren medizinischen Verschlussvorrichtungen (130) eine erste medizinische Verschlussvorrichtung (132) mit einem proximalen Ende, das neben dem distalen Ende (116) des länglichen Schafts (110) angeordnet ist, und eine zweite medizinische Verschlussvorrichtung (134) mit einem proximalen Ende, das neben einem distalen Ende der ersten medizinischen Verschlussvorrichtung (132) angeordnet ist, aufweisen.

3. Gefäßverschlusssystem nach Anspruch 2, ferner aufweisend einen ersten Befestigungsmechanismus (170), der zwischen dem distalen Ende (116) des länglichen Schafts (110) und dem proximalen Ende der ersten medizinischen Verschlussvorrichtung (132) angeordnet ist;
wobei der erste Befestigungsmechanismus (170) aufweist:
ein erstes Teil (172) mit einem ersten Längslumen (174), das zum verschiebbaren Aufnehmen des Freigabedrahts (120) konfiguriert ist, und ein zweites Teil (176) mit einem zweiten Längslumen (178), das zum verschiebbaren Aufnehmen des Freigabedrahts (120) konfiguriert ist;
wobei das erste Teil (172) fest an dem distalen Ende (116) des länglichen Schafts (110) und das zweite Teil (176) fest an dem proximalen Ende der ersten medizinischen Verschlussvorrichtung (132) angebracht ist;
wobei das erste Teil (172) und das zweite Teil (176) so zum Ineinandergreifen konfiguriert sind, dass eine relative axiale Verschiebung zwischen dem ersten Teil (172) und dem zweiten Teil (176) verhindert wird, wenn das erste Teil (172) an das zweite Teil (176) anstößt und das erste Längslumen (174) koaxial mit dem zweiten Längslumen (178) ausgerichtet ist.

4. Gefäßverschlusssystem nach Anspruch 3, wobei das erste Teil (172) und das zweite Teil (176) so zum Ineinandergreifen konfiguriert sind, dass eine relative seitliche Verschiebung zwischen dem ersten Teil (172) und dem zweiten Teil (176) verhindert wird, wenn das erste Teil (172) an das zweite Teil (176) anstößt, das erste Längslumen (174) koaxial mit dem zweiten Längslumen (178) ausgerichtet ist und der Freigabedraht (120) verschiebbar mit dem ersten Längslumen (174) und dem zweiten Längslumen (178) in Eingriff steht.

5. Gefäßverschlusssystem nach Anspruch 2, ferner aufweisend einen zweiten Befestigungsmechanismus (180), der zwischen dem distalen Ende der ersten medizinischen Verschlussvorrichtung (132) und dem proximalen Ende der zweiten medizinischen Verschlussvorrichtung (134) angeordnet ist;
wobei der zweite Befestigungsmechanismus (180) aufweist:
ein drittes Teil (182) mit einem dritten Längslumen (184), das zum verschiebbaren Aufnehmen des Freigabedrahts (120) konfiguriert ist, und ein viertes Teil (186) mit einem vierten Längslumen (188), zum verschiebbaren Aufnehmen des Freigabedrahts (120) konfiguriert ist;
wobei das dritte Teil (182) fest an dem distalen Ende der ersten medizinischen Verschlussvorrichtung (132) und das vierte Teil (186) fest an dem proximalen Ende der zweiten medizinischen Verschlussvorrichtung (134) angebracht ist;
wobei das dritte Teil (182) und das vierte Teil (186) so zum Ineinandergreifen konfiguriert sind, dass eine relative axiale Verschiebung zwischen dem dritten Teil (182) und dem vierten Teil (186) verhindert wird, wenn das dritte Teil (182) an das vierte Teil (186) anstößt und das dritte Längslumen (184) koaxial mit dem vierten Längslumen (188) ausgerichtet ist.

6. Gefäßverschlusssystem nach Anspruch 5, wobei das dritte Teil (182) und das vierte Teil (186) so zum Ineinandergreifen konfiguriert sind, dass eine relative seitliche Verschiebung zwischen dem dritten Teil (182) und dem vierten Teil (186) verhindert wird, wenn das dritte Teil (182) an das vierte Teil (186) anstößt, das dritte Längslumen (184) koaxial mit dem vierten Längslumen (188) ausgerichtet ist und der Freigabedraht (120) verschiebbar mit dem dritten Längslumen (184) und dem vierten Längslumen (188) in Eingriff steht.

7. Gefäßverschlusssystem nach Anspruch 2, wobei die mehreren medizinischen Verschlussvorrichtungen (130) eine dritte medizinische Verschlussvorrichtung (136) mit einem proximalen Ende aufweisen, das neben einem distalen Ende der zweiten medizinischen Verschlussvorrichtung (134) angeordnet ist.

8. Gefäßverschlusssystem nach Anspruch 7, ferner aufweisend einen dritten Befestigungsmechanismus (190), der zwischen dem distalen Ende der zweiten medizinischen Verschlussvorrichtung (134) und dem proximalen Ende der dritten medizinischen Verschlussvorrichtung (136) angeordnet ist;
wobei der dritte Befestigungsmechanismus (190) aufweist:
ein fünftes Teil (192) mit einem fünften Längslumen (194), das zum verschiebbaren Aufnehmen des Freigabedrahts (120) konfiguriert ist, und ein sechstes Teil (196) mit einem sechsten Längslumen (198), das zum verschiebbaren Aufnehmen des Freigabedrahts (120) konfiguriert ist;
wobei das fünfte Teil (192) fest an dem distalen Ende der zweiten medizinischen Verschlussvorrichtung (134) und das sechste Teil (196) fest an dem proximalen Ende der dritten medizinischen Verschlussvorrichtung (136) angebracht ist;
wobei das fünfte Teil (192) und das sechste Teil (196) so zum Ineinandergreifen konfiguriert sind, dass eine relative axiale Verschiebung zwischen dem fünften Teil (192) und dem sechsten Teil (196) verhindert wird, wenn das fünfte Teil (192) an das sechste Teil (196) anstößt und das fünfte Längslumen (194) koaxial mit dem sechsten Längslumen (198) ausgerichtet ist.

9. Gefäßverschlusssystem nach Anspruch 8, wobei das fünfte Teil (192) und das sechste Teil (196) so zum Ineinandergreifen konfiguriert sind, dass eine relative seitliche Verschiebung zwischen dem fünften Teil (192) und dem sechsten Teil (196) verhindert wird, wenn das fünfte Teil (192) an das sechste Teil (196) anstößt, das fünfte Längslumen (194) koaxial mit dem sechsten Längslumen (198) ausgerichtet ist und der Freigabedraht (120) verschiebbar mit dem fünften Längslumen (194) und dem sechsten Längslumen (198) in Eingriff steht.

10. Gefäßverschlusssystem nach einem der Ansprüche 1 bis 9, wobei mindestens eine der mehreren medizinischen Verschlussvorrichtungen (130) ein gewendeltes Element ist, das zum Annehmen einer ersten Form, wenn es mit dem länglichen Schaft (110) verbunden ist, und einer zweiten Form, wenn es von dem länglichen Schaft (110) getrennt ist, konfiguriert ist.

11. Gefäßverschlusssystem nach einem der Ansprüche 1-10,
wobei mindestens eine der mehreren medizinischen Verschlussvorrichtungen (130) eine andere Länge als eine andere der mehreren medizinischen Verschlussvorrichtungen (130) aufweist.

12. Gefäßverschlusssystem nach Anspruch 1, ferner aufweisend einen zweiten Freigabedraht (122), der verschiebbar innerhalb des länglichen Schafts (110) und mindestens eines Teils jeder der mehreren medizinischen Verschlussvorrichtungen (130) angeordnet ist, wobei der zweite Freigabedraht (122) eine Kugelspitze (124) aufweist.

13. Gefäßverschlusssystem nach Anspruch 12, ferner aufweisend ein Sicherungselement (126), das fest an einem proximalen Ende jeder der mehreren medizinischen Verschlussvorrichtungen (130) angebracht ist, wobei dann, wenn sich der Freigabedraht (120) und der zweite Freigabedraht (122) beide gleichzeitig durch das Sicherungselement (126) erstrecken, der zweite Freigabedraht (122) daran gehindert wird, durch das Sicherungselement (126) zurückgezogen zu werden.

## Revendications

1. Système d'occlusion vasculaire (100) comprenant :
un microcathéter (200) configuré pour circuler dans un système vasculaire ;
une tige allongée (110) disposée, de manière coulissante, à l'intérieur d'une lumière (202) du microcathéter (200), la tige allongée (110) ayant une lumière (112) s'étendant à partir d'une extrémité proximale (114) de la tige allongée (110) à une extrémité distale (116) de la tige allongée (110) ;
une pluralité de dispositifs médicaux occlusifs (130) raccordés, de manière amovible, à la tige allongée (110),
dans lequel au moins l'un de la pluralité de dispositifs médicaux occlusifs (130) a une rigidité différente d'un dispositif différent de la pluralité de dispositifs médicaux occlusifs (130),
dans lequel le dispositif le plus distal de la pluralité de dispositifs médicaux occlusifs (130) a une rigidité supérieure à l'un quelconque de la pluralité de dispositifs médicaux occlusifs ; et
un fil de libération (120) disposé, de manière coulissante, à l'intérieur de la tige allongée (110) et d'au moins une partie de chacun de la pluralité de dispositifs médicaux occlusifs (130) ;
dans lequel le fil de libération (120) fixe chacun de la pluralité de dispositifs médicaux occlusifs (130) à l'extrémité distale (116) de la tige allongée (110), lorsque le fil de libération (120) est disposé dans au moins une partie de chacun de la pluralité de dispositifs médicaux occlusifs (130),
dans lequel le fil de libération (120) comprend un ou plusieurs indicateurs (128) disposés à proximité de l'extrémité proximale du fil de libération (120) configurée pour montrer la quantité du fil de libération (120) qui a été retirée et/ou configurée pour communiquer à un utilisateur du système d'occlusion vasculaire (100) la quantité de la pluralité de dispositifs médicaux occlusifs (130) qui a été libérée.

2. Système d'occlusion vasculaire selon la revendication 1, dans lequel la pluralité de dispositifs médicaux occlusifs (130) comprend un premier dispositif médical occlusif (132) ayant une extrémité proximale disposée de manière adjacente à l'extrémité distale (116) de la tige allongée (110) et un deuxième dispositif médical occlusif (134) ayant une extrémité proximale disposée de manière adjacente à une extrémité distale du premier dispositif médical occlusif (132).

3. Système d'occlusion vasculaire selon la revendication 2, comprenant en outre un premier mécanisme de fixation (170) disposé entre l'extrémité distale (116) de la tige allongée (110) et l'extrémité proximale du premier dispositif médical occlusif (132) ;
dans lequel le premier mécanisme de fixation (170) comprend :
une première partie (172) ayant une première lumière longitudinale (174) configurée pour recevoir, de manière coulissante, le fil de libération (120), et une deuxième partie (176) ayant une deuxième lumière longitudinale (178) configurée pour recevoir, de manière coulissante, le fil de libération (120) ;
dans lequel la première partie (172) est fixée, de manière fixe, à l'extrémité distale (116) de la tige allongée (110) et la deuxième partie (176) est fixée, de manière fixe, sur l'extrémité proximale du premier dispositif médical occlusif (132) ;
dans lequel la première partie (172) et la deuxième partie (176) sont configurées pour s'imbriquer l'une dans l'autre de sorte que la translation axiale relative entre la première partie (172) et la deuxième partie (176) est empêchée lorsque la première partie (172) vient en butée contre la deuxième partie (176) et que la première lumière longitudinale (174) est alignée, de manière coaxiale, avec la deuxième lumière longitudinale (178).

4. Système d'occlusion vasculaire selon la revendication 3, dans lequel la première partie (172) et la deuxième partie (176) sont configurées pour s'imbriquer l'une dans l'autre de sorte que la translation latérale relative entre la première partie (172) et la deuxième partie (176) est empêchée lorsque la première partie (172) vient en butée contre la deuxième partie (176), que la première lumière longitudinale (174) est alignée de manière coaxiale avec la deuxième lumière longitudinale (178), et que le fil de libération (120) est mis en prise, de manière coulissante, avec la première lumière longitudinale (174) et la deuxième lumière longitudinale (178).

5. Système d'occlusion vasculaire selon la revendication 2, comprenant en outre un deuxième mécanisme de fixation (180) disposé entre l'extrémité distale du premier dispositif médical occlusif (132) et l'extrémité proximale du deuxième dispositif médical occlusif (134) ;
dans lequel le deuxième mécanisme de fixation (180) comprend :
une troisième partie (182) ayant une troisième lumière longitudinale (184) configurée pour recevoir, de manière coulissante, le fil de libération (120), et une quatrième partie (186) ayant une quatrième lumière longitudinale (188) configurée pour recevoir, de manière coulissante, le fil de libération (120) ;
dans lequel la troisième partie (182) est fixée, de manière fixe, à l'extrémité distale du premier dispositif médical occlusif (132) et la quatrième partie (186) est fixée, de manière fixe, à l'extrémité proximale du deuxième dispositif médical occlusif (134) ;
dans lequel la troisième partie (182) et la quatrième partie (186) sont configurées pour s'imbriquer l'une dans l'autre de sorte que la translation axiale relative entre la troisième partie (182) et la quatrième partie (186) est empêchée lorsque la troisième partie (182) vient en butée contre la quatrième partie (186) et que la troisième lumière longitudinale (184) est alignée, de manière coaxiale, avec la quatrième lumière longitudinale (188).

6. Système d'occlusion vasculaire selon la revendication 5, dans lequel la troisième partie (182) et la quatrième partie (186) sont configurées pour s'imbriquer l'une dans l'autre de sorte que la translation latérale relative entre la troisième partie (182) et la quatrième partie (186) est empêchée, lorsque la troisième partie (182) vient en butée contre la quatrième partie (186), que la troisième lumière longitudinale (184) est alignée de manière coaxiale avec la quatrième lumière longitudinale (188) et que le fil de libération (120) est mis en prise, de manière coulissante, avec la troisième lumière longitudinale (184) et la quatrième lumière longitudinale (188).

7. Système d'occlusion vasculaire selon la revendication 2, dans lequel la pluralité de dispositifs médicaux occlusifs (130) comprend un troisième dispositif médical occlusif (136) ayant une extrémité proximale disposée de manière adjacente à une extrémité distale du deuxième dispositif médical occlusif (134).

8. Système d'occlusion vasculaire selon la revendication 7, comprenant en outre un troisième mécanisme de fixation (190) disposé entre l'extrémité distale du deuxième dispositif médical occlusif (134) et l'extrémité proximale du troisième dispositif médical occlusif (136) ;
dans lequel le troisième mécanisme de fixation (190) comprend :
une cinquième partie (192) ayant une cinquième lumière longitudinale (194) configurée pour recevoir, de manière coulissante, le fil de libération (120) et une sixième partie (196) ayant une sixième lumière longitudinale (198) configurée pour recevoir, de manière coulissante, le fil de libération (120) ;
dans lequel la cinquième partie (192) est fixée, de manière fixe, à l'extrémité distale du deuxième dispositif médical occlusif (134) et la sixième partie (196) est fixée, de manière fixe, à l'extrémité proximale du troisième dispositif médical occlusif (136) ;
dans lequel la cinquième partie (192) et la sixième partie (196) sont configurées pour s'imbriquer l'une dans l'autre de sorte que la translation axiale relative entre la cinquième partie (192) et la sixième partie (196) est empêchée lorsque la cinquième partie (192) vient en butée contre la sixième partie (196) et que la cinquième lumière longitudinale (194) est alignée, de manière coaxiale, avec la sixième lumière longitudinale (198).

9. Système d'occlusion vasculaire selon la revendication 8, dans lequel la cinquième partie (192) et la sixième partie (196) sont configurées pour s'imbriquer l'une dans l'autre de sorte que la translation latérale relative entre la cinquième partie (192) et la sixième partie (196) est empêchée lorsque la cinquième partie (192) vient en butée contre la sixième partie (196), que la cinquième lumière longitudinale (194) est alignée, de manière coaxiale, avec la sixième lumière longitudinale (198), et que le fil de libération (120) est mis en prise, de manière coulissante, avec la cinquième lumière longitudinale (194) et la sixième lumière longitudinale (198).

10. Système d'occlusion vasculaire selon l'une quelconque des revendications 1 à 9, dans lequel au moins l'un de la pluralité de dispositifs médicaux occlusifs (130) est un élément hélicoïdal configuré pour adopter une première forme lorsqu'il est raccordé à la tige allongée (110) et une deuxième forme lorsqu'il est déconnecté de la tige allongée (110).

11. Système d'occlusion vasculaire selon l'une quelconque des revendications 1 à 10, dans lequel au moins l'un de la pluralité de dispositifs médicaux occlusifs (130) a une longueur différente d'un dispositif différent de la pluralité de dispositifs médicaux occlusifs (130).

12. Système d'occlusion vasculaire selon la revendication 1, comprenant en outre un deuxième fil de libération (122) disposé, de manière coulissante, à l'intérieur de la tige allongée (110) et d'au moins une partie de chacun de la pluralité de dispositifs médicaux occlusifs (130), dans lequel le deuxième fil de libération (122) comprend une pointe sphérique (124).

13. Système d'occlusion vasculaire selon la revendication 12, comprenant en outre un élément de fixation (126) fixé, de manière fixe, à une extrémité proximale de chacun de la pluralité de dispositifs médicaux occlusifs (130), dans lequel lorsque le fil de libération (120) et le deuxième fil de libération (122) s'étendent tous deux à travers l'élément de fixation (126) simultanément, on empêche le deuxième fil de libération (122) d'être retiré par l'élément de fixation (126).
